# FASCICULE DE BREVET EUROPEEN

(11) **EP 0 494 474 B1**
(45) Date de publication et mention de la délivrance du brevet: **21.09.1994**
(21) Numéro de dépôt: 91203419.6
(22) Date de dépôt: 30.12.1991
(51) Int. Cl.: B01J 27/138, B01J 27/122, B01J 27/10, C07C 17/156

(54) **Composition catalytique pour l'oxychloration et procédé d'oxychloration de l'éthylène utilisant une telle composition**
Oxychlorierung katalytischer Zusammensetzung und Verfahren für die Oxychlorierung von Ethylen unter Verwendung dieser Zusammensetzung
Oxychlorination catalytic composition and ethylene oxychlorination process using this composition

(30) Priorité: 11.01.1991 BE 9100025; 10.07.1991 BE 9100659
(43) Date de publication de la demande: 15.07.1992
(73) Titulaire: SOLVAY (Société Anonyme), B-1050 Bruxelles (BE)
(72) Inventeur: Derleth, Helmut, W-3070 Nienburg (DE); Strebelle, Michel, B-1150 Bruxelles (BE)
(74) Mandataire: Marckx, Frieda

(56) Documents cités:
- EP-A- 0 206 265
- EP-A- 0 255 156
- EP-A- 0 375 202
- US-A- 4 194 990
- US-A- 5 004 849

## Description

La présent invention se rapporte à une composition catalytique pour l'oxychloration et à un procédé d'oxychloration de l'éthylène utilisant une telle composition catalytique.

L'oxychloration, c'est-à-dire la chloration d'hydrocarbures par du chlorure d'hydrogène en présence d'air ou d'oxygène, constitue une réaction connue de longue date qui s'effectue habituellement en présence de catalyseurs constitués par des sels métalliques déposés sur des supports inertes tels que des alumines, des gels de silice, des oxydes mixtes ou encore des argiles ou autres supports d'origine naturelle. Industriellement, le catalyseur est utilisé le plus fréquemment en lit fluide mais peut aussi être employé en lit fixe. Comme sels métalliques, on utilise le plus souvent des halogénures tels que le chlorure de cuivre. Lorsqu'il est utilisé seul, le chlorure de cuivre présente toutefois l'inconvénient d'être relativement volatil, ce qui entraîne une chute de l'activité catalytique et du rendement de la réaction d'oxychloration, inacceptable dans les installations industrielles.

Il est bien connu d'améliorer les performances des catalyseurs d'oxychloration constitués de chlorure de cuivre sur support par ajout de chlorures de métaux alcalins, de métaux alcalino-terreux ou de terres rares (lanthanides). L'addition de chlorures de métaux alcalins est réputée augmenter la sélectivité de la réaction, notamment en limitant les réactions parallèles d'oxydation de l'éthylène en CO et CO₂. Le chlorure de magnésium est utilisé notamment en vue d'améliorer la fluidisation du catalyseur.

Des compositions catalytiques pour l'oxychloration comprenant simultanément des chlorures de cuivre, de magnésium et de métaux alcalins sur support inerte ont déjà été proposées.

Le brevet US-A-3624170 (TOYO SODA) décrit une composition catalytique ternaire à base des chlorures de cuivre, de sodium et de magnésium (en des quantités spécifiques), laquelle permettrait d'éviter en outre la désactivation causée par la contamination de Fe₂Cl₆ qui serait présent lorsque des réacteurs en acier inoxydable sont utilisés.

La demande EP-A-0375202 (ICI) décrit une composition catalytique ternaire à base des chlorures de cuivre, de magnésium et de potassium, mis en oeuvre en proportions telles que le rapport atomique Cu/Mg/K est, de préférence, 1 / 0,2-0,9 / 0,2-0,9.

La demande EP-A-0255156 (SOLVAY) décrit des compositions catalytiques ternaires contenant un mélange des chlorures de cuivre, de magnésium et d'un métal alcalin choisi parmi le sodium ou le lithium utilisés dans des proportions précises qui permettent d'atteindre un très bon rendement dans un procédé en lit fluide d'oxychloration de l'éthylène en 1,2-dichloréthane, en réduisant simultanément la corrosion des réacteurs en acier inoxydable, grâce, notamment à une réduction du collage et du mottage des grains de catalyseur. Ce document enseigne que, pour les compositions ternaires contenant les chlorures de cuivre, de magnésium et de sodium à titre de métal alcalin, un rapport atomique Na/Cu supérieur à 0,2/1 conduit à des problèmes de corrosion du réacteur. Par contre, si le lithium est utilisé à titre de métal alcalin, aucun phénomène de corrosion n'apparaît dans une large gamme de rapports atomiques Li/Cu. Cependant, les exemples montrent l'apparition de problèmes de collage et de mottage du catalyseur avec les compositions contenant du lithium dans un rapport Li/Cu supérieur à 0,6.

Il a récemment été observé que même les compositions apparemment fluidisables et stables de l'art antérieur présentent, après quelques mois d'exploitation industrielle, une tendance au collage sur la paroi du réacteur, ce qui constitue un obstacle important à leur utilisation, alors que ces compositions catalytiques ternaires offrent par ailleurs d'excellentes performances au point de vue de l'activité et de la sélectivité de la réaction d'oxychloration. Toute diminution de la quantité de chlorure de métal alcalin dans la composition catalytique ternaire en vue d'éviter ces problèmes de collage provoque malencontreusement une chute de la sélectivité et donc, du rendement en 1,2-dichloréthane.

On a maintenant trouvé une composition catalytique particulièrement performante qui permet d'atteindre un rendement élevé sans plus rencontrer de phénomènes de collage ou de mottage du catalyseur, ni de corrosion du réacteur. Cette composition catalytique convient particulièrement bien pour les procédés d'oxychloration en lit fluide.

La présente invention concerne une composition catalytique d'oxychloration comprenant des chlorures de cuivre, de magnésium et de lithium déposés sur un support inerte, qui se caractérise en ce qu'elle contient en outre au moins un chlorure de métal alcalin autre que le lithium, en ce qu'elle présente une teneur en cuivre, calculée sous forme métallique par rapport au poids total de la composition catalytique, de 30 à 90 g/kg, et en ce que les différents chlorures de métaux sont présents dans des rapports atomiques Cu : Mg : Li : autre(s) métal(aux) alcalin(s) de 1 : 0,1-1,5 : 0,01-1,0 : 0,001-0,8. Elle concerne aussi un procédé d'oxychloration de l'éthylène en 1,2-dichloréthane caractérisé en ce que la réaction d'oxychloration est catalysée par la composition catalytique selon l'invention.

De manière surprenante, il a été observé que des compositions catalytiques contenant, en plus des chlorures de cuivre, de magnésium et de lithium, au moins un chlorure de métal alcalin autre que le lithium permettent d'atteindre un rendement encore jamais obtenu sans rencontrer les inconvénients exposés ci-dessus avec des compositions ternaires de l'art antérieur.

A titre d'exemples de chlorures d'autres métaux alcalins utilisables, on peut mentionner les chlorures de sodium, de potassium, de rubidium et de césium. On utilise néanmoins de préférence le chlorure de sodium, le chlorure de potassium et les mélanges de chlorure de sodium et de chlorure de potassium.

Les compositions catalytiques selon l'invention présentent une teneur en cuivre, calculée sous forme métallique par rapport au poids total de la composition catalytique, le plus souvent d'au moins 40 g/kg. Le plus souvent, elle ne dépasse pas 70 g/kg. De préférence, la quantité de chlorure de cuivre est d'au moins 50 g/kg. De préférence, elle ne dépasse pas 65 g/kg.

La teneur des autres sels dans les compositions catalytiques peut aisément être déduite des teneurs en cuivre ainsi spécifiées, en combinaison avec les rapports atomiques précisés ci-après.

Les proportions (exprimées en rapport atomique des métaux) dans lesquelles les chlorures de cuivre, de magnésium, de lithium et de l'autre ou des autres métaux alcalins sont mis en oeuvre sont définies comme suit.
- Le rapport atomique Mg/Cu est d'au moins 0,1. Il est de préférence d'au moins 0,3. Ce rapport ne dépasse pas 1,5. Dans les compositions préférées, ce rapport Mg/Cu ne dépasse pas 1,0.
- Le rapport atomique Li/Cu est d'au moins 0,01. Il est de préférence d'au moins 0,1. Dans les conditions les plus particulièrement préférées, il est d'au moins 0,15. Ce rapport ne dépasse pas 1,0. Dans les compositions préférées, ce rapport Li/Cu ne dépasse pas 0,8. Dans les conditions les plus particulièrement préférées, il ne dépasse pas 0,6.
- Le rapport atomique entre chacun des métaux alcalins autre que le lithium et le cuivré est d'au moins 0,001. Il est de préférence d'au moins 0,01. Dans les conditions les plus particulièrement préférées, il est d'au moins 0,03. Ce rapport ne dépasse pas 0,8. Dans les compositions préférées, il ne dépasse pas 0,6. Dans les conditions les plus particulièrement préférées, il ne dépasse pas 0,4.

Les compositions catalytiques préférées selon l'invention sont donc caractérisées par des rapports atomiques Cu : Mg : Li : autre(s) métal(aux) alcalin(s) de 1 : 0,3-1,0 : 0,1-0,8 : 0,01-0,6.

Suivant un mode de réalisation préféré de la présente invention, le rapport atomique entre la somme de tous les métaux alcalins (y compris le lithium) et le cuivre ne dépasse pas 1,5. Dans les conditions particulièrement préférées, ce rapport ne dépasse pas 1,2. Dans les conditions les plus particulièrement préférées, il ne dépasse pas 0,8.

De préférence, le rapport atomique entre chacun des métaux alcalins autre que le lithium et le lithium, est compris entre 0,1 et 2,0. De manière particulièrement préférée, ce rapport est compris entre 0,2 et 1,5.

Le support utilisé pour les compositions catalytiques selon l'invention est choisi parmi des supports inertes tels que des alumines, des gels de silice, des oxydes mixtes ou encore des argiles ou autres supports d'origine naturelle. De préférence, le support pour les compositions catalytiques de l'invention est une alumine. L'alumine mise en oeuvre dans les compositions catalytiques de l'invention peut être de toute origine et être obtenue selon tout procédé connu; on utilise habituellement des alumines de type η ou γ. De bons résultats ont été obtenus avec une alumine γ.

L'alumine généralement mise en oeuvre dans les compositions catalytiques de l'invention, présente un diamètre moyen des particules compris entre 10 et 200 »m et de préférence un diamètre moyen compris entre 20 et 120 »m.

La surface spécifique de l'alumine mesurée suivant la méthode B.E.T. est généralement comprise entre 50 m²/g et 250 m²/g. De bons résultats ont été obtenus avec une alumine dont la surface spécifique était comprise entre 100 m²/g et 210 m²/g.

Enfin, le volume poreux des alumines habituellement utilisées se situe entre 0,1 et 1 cm³/g. De préférence, le volume poreux est compris entre 0,2 et 0,8 cm³/g et de bons résultats ont été obtenus avec une alumine dont le volume poreux se situe entre 0,3 et 0,6 cm³/g.

Il est à noter que l'alumine, de par sa nature et son mode de synthèse, contient une quantité plus ou moins grande d'atomes de métaux alcalins, notamment de sodium, pouvant être intégrés dans le réseau cristallin ou être liés sous une forme chimique quelconque. Selon la nature de l'alumine, celle-ci peut contenir classiquement de 20 à 5000 ppm de métaux alcalins. La présence de ces atomes de métaux alcalins que l'on peut plus aisément qualifier de "non lavables", n'entre pas en ligne de compte pour la détermination de la teneur en métaux alcalins dans les compositions catalytiques selon la présente invention, qui se rapporte uniquement aux métaux alcalins ajoutés sous forme de sels pour former la composition catalytique et pouvant être considérés comme constituant des "métaux alcalins lavables". Ces sels alcalins ne sont pas liés chimiquement au support d'alumine et sont généralement introduits dans les compositions catalytiques par imprégnation de l'alumine avec les sels considérés. Cette imprégnation avec les sels alcalins désirés peut se faire, soit en même temps que l'imprégnation avec les autres sels, soit avant, soit après cette imprégnation.

Le mode d'obtention des compositions catalytiques selon l'invention n'est pas critique.

Les chlorures métalliques peuvent être introduits dans la composition catalytique soit directement sous forme de chlorures, par exemple par imprégnation du support à l'aide d'une solution renfermant un mélange de ces sels, soit sous forme d'autres composés des métaux, tels que les oxydes, les hydroxydes, les nitrates ou tout autre composé capable d'être transformé en chlorure dans les conditions dans lesquelles sont menées les réactions d'oxychloration.

Un mode d'obtention ayant donné de bons résultats consiste à imprégner une alumine avec une solution aqueuse renfermant les quantités adéquates des chlorures de cuivre, de magnésium, de lithium et des autres métaux alcalins désirés. On évite l'apparition d'une phase liquide non adsorbée par le solide en limitant le volume de la solution imprégnante à 70 à 100 % du volume poreux de la quantité d'alumine mise en oeuvre. Cette alumine imprégnée est ensuite séchée avant de l'introduire dans le réacteur d'oxychloration proprement dit.

Un autre mode de préparation utilisable pour préparer des compositions catalytiques selon l'invention consiste à mélanger deux compositions catalytiques différentes, par exemple une composition ternaire contenant du chlorure de cuivre, du chlorure de magnésium et du chlorure de lithium avec une composition catalytique binaire à base de chlorure de cuivre et de chlorure de potassium, en proportions telles que l'on obtienne globalement une composition catalytique selon l'invention contenant les chlorures de cuivre, de magnésium, de lithium et de potassium avec les quantités voulues des différents chlorures métalliques et les rapports atomiques souhaités. D'autres mélanges de compositions catalytiques différentes peuvent être réalisés.

Les compositions catalytiques finales présentent généralement une surface spécifique B.E.T. comprise entre 25 m²/g et 200 m²/g et de préférence entre 50 et 150 m²/g. De bons résultats ont été obtenus avec des compositions catalytiques dont la surface spécifique B.E.T. est comprise entre 80 et 140 m²/g.

Les compositions catalytiques sont particulièrement avantageuses dans un procédé d'oxychloration dans lequel le catalyseur est sous forme de lit fluidisé, du fait d'un rendement amélioré. Elles peuvent aussi être mises en oeuvre dans un procédé d'oxychloration réalisé avec un catalyseur disposé en lit fixe, moyennant mise en forme appropriée des particules de catalyseur, par exemple sous forme de granules de quelques mm de diamètre.

L'oxygène moléculaire nécessaire à la réaction d'oxychloration est introduit dans le réacteur, soit dilué, par exemple sous forme d'air, soit pur. Par oxygène pur, on entend essentiellement non dilué. L'emploi d'oxygène pur est particulièrement avantageux, car il permet de recycler dans le réacteur les réactifs non convertis et il limite la quantité des gaz à traiter à la sortie du réacteur.

Les compositions catalytiques selon l'invention conviennent tout particulièrement pour un procédé d'oxychloration de l'éthylène en 1,2-dichloréthane dans lequel le catalyseur est sous forme de lit fluidisé et dans lequel l'oxygène est introduit sous forme pure.

Lorsque l'on opère avec un catalyseur disposé en lit fluidisé, la température à laquelle s'effectue la réaction d'oxychloration se situe habituellement entre 200 et 300 °C. De préférence, cette température est comprise entre 220 et 280 °C. Enfin, des bons résultats ont été obtenus avec une température située aux environs de 240 °C-270 °C.

La pression à laquelle est effectuée la réaction d'oxychloration n'est pas critique en elle-même. Habituellement, on opère avec des pressions comprises entre 0,1 et 1 MPa et de préférence avec des pressions comprises entre 0,1 et 0,8 MPa.

La vitesse de fluidisation des compositions catalytiques n'est pas critique en elle-même et dépend essentiellement de la granulométrie du catalyseur et des dimensions de l'appareillage. Généralement, on opère avec des vitesses comprises entre 5 et 100 cm/s.

Enfin, le rapport des réactifs mis en oeuvre est le même que celui généralement utilisé dans les procédés antérieurs. D'habitude, on opère avec un léger excès d'éthylène par rapport à la quantité d'HCl mise en oeuvre. Toutefois, les compositions catalytiques de l'invention permettent également de travailler au voisinage de la stoechiométrie, voire même en excès d'HCl.

L'invention se trouve plus amplement illustrée par les exemples suivants.
Les exemples annotés (c) se rapportent à des exemples donnés à titre comparatif.

### Exemples 1 à 8

Un échantillon d'une composition catalytique correspondant à l'exemple 1(c) est préparé au départ d'une alumine gamma dont les caractéristiques sont les suivantes : surface spécifique = 186 m²/g; volume poreux = 0,38 cm³/g; poids spécifique par écoulement libre de 0,75 kg/dm³; diamètre moyen des particules = 50 »m. A environ 800 g de cette alumine est ajoutée une solution aqueuse d'imprégnation comprenant à l'état dissous 162 g de CuCl₂.2H₂O, 143 g de MgCl₂.6H₂O et 21 g de LiCl. Le solide humide est ensuite chauffé à 150 °C. 1 kg de catalyseur est ainsi obtenu avec, calculée sous forme métallique par rapport au poids total du catalyseur, une teneur en cuivre de 60 g/kg, une teneur en magnésium de 17 g/kg et une teneur en lithium de 3,3 g/kg. Exprimée en rapport atomique, la proportion des différents métaux Cu : Mg : Li est de 1 : 0,74 : 0,50.

Les exemples 2(c), 3(c) et 4(c) sont préparés de la même manière, au départ de la même alumine imprégnée par des solutions aqueuses renfermant les trois chlorures métalliques désirés, CuCl₂.2H₂O, MgCl₂.6H₂O et NaCl ou KCl, en quantités et proportions adéquates.

Les exemples 5 à 7, selon l'invention, sont préparés de la même manière, au départ de la même alumine imprégnée par une solution aqueuse renfermant CuCl₂.2H₂O, MgCl₂.6H₂O, LiCl, et KCl ou NaCl en quantités et proportions adéquates.

L'exemple 8 est préparé par mélange mécanique de deux compositions ternaires contenant l'une des chlorures de cuivre, de magnésium et de lithium et l'autre des chlorures de cuivre, de magnésium et de sodium.

Le tableau 1 ci-après reprend les teneurs de ces différentes compositions catalytiques.

**TABLEAU 1**

| Numéros des Exemples | Teneur en métaux (g/kg) | | | | | Proportions atomiques des métaux | | | | |
|---|---|---|---|---|---|---|---|---|---|---|
| | Cu | Mg | Li | Na | K | Cu | Mg | Li | Na | K |
| 1(c) | 60 | 17 | 3,3 | - | - | 1 | 0,74 | 0,50 | - | - |
| 2(c) | 60 | 17 | - | 6,2 | - | 1 | 0,74 | - | 0,29 | - |
| 3(c) | 58 | 17 | - | - | 11 | 1 | 0,77 | - | - | 0,31 |
| 4(c) | 59 | 17 | - | - | 17 | 1 | 0,75 | - | - | 0,47 |
| 5 | 57 | 16 | 1,6 | - | 12 | 1 | 0,73 | 0,26 | - | 0,34 |
| 6 | 60 | 17 | 2,0 | 2,7 | - | 1 | 0,74 | 0,31 | 0,12 | - |
| 7 | 56 | 16 | 3,0 | 2,0 | - | 1 | 0,75 | 0,49 | 0,10 | - |
| 8 | 48 | 13,5 | 1,3 | 0,8 | - | 1 | 0,74 | 0,25 | 0,05 | - |

### Exemples 9 à 16

225 cm³ des compositions catalytiques décrites dans les exemples 1 à 8, sont disposés dans un réacteur en Inconel 600, d'oxychloration de l'éthylène en lit fluide en 1,2-dichloréthane (DCEa)

Dans ce réacteur, les gaz réactifs sont introduits par le bas à travers un filtre métallique fritté. Les conditions opératoires dans lesquelles sont réalisés ces exemples sont les suivantes :
- débit des réactifs (lN/h) : C₂H₄ / HCl / Air : 84 / 160 / 260
- température : 255 °C
- pression : 0,6 MPa
- vitesse de fluidisation : 10 cm/s
- temps de contact : 5 s
Les rendements obtenus dans ces essais de laboratoire sont limités par le temps de séjour restreint de 5 s. La conversion de C₂H₄ est, dès lors, limitée dans tous les essais (les conditions de marche d'un réacteur industriel permettent d'atteindre des temps de séjour nettement plus longs : 10 à 80 s, et le plus souvent de 20 à 50 s afin d'assurer une meilleure conversion de l'éthylène). Les résultats de sélectivité obtenus sont cependant tout à fait significatifs pour comparer les performances des différentes compositions catalytiques.

Les produits de réaction sont ensuite détendus jusqu'à la pression atmosphérique par une vanne de régulation de pression du réacteurs. Les produits de réaction sont ensuite refroidis dans un piège maintenu à - 20 °C et les gaz non condensés sont lavés dans un scrubber à eau avant de balayer une ampoule de prélèvement. Le bilan des produits formés est effectué au départ d'analyses chromatographiques des produits liquide et gazeux recueillis et du titrage de l'acidité de la solution aqueuse recueillie au pied du scrubber.

La tendance au collage des compositions catalytiques est mesurée dans un réacteur micro-pilote fonctionnant dans les mêmes conditions opératoires que le réacteur décrit ci-dessus, mais muni d'un tube interne (doigt de gant) parcouru par une huile maintenue à une température inférieure à la température à laquelle est menée la réaction. La tendance au collage est déterminée visuellement par examen de ce tube interne après 20 heures de fonctionnement.

Les résultats des exemples 9 à 16 correspondant aux 8 essais réalisés avec les compositions catalytiques 1 à 8 sont rassemblés au Tableau 2.

**TABLEAU 2**

| Numéros des Exemples | Origine des compositions catalytiques | Rendement DCEa par rapport à HCl (% mol) | Sélectivité par rapport à l'éthylène converti (% molaire) | | | Tendance au collage |
|---|---|---|---|---|---|---|
| | | | DCEa | EtCl | CO+CO2 | |
| 9(c) | Ex.1(c) | 96,6 | 98,0 | 0,3 | 1,0 | OUI |
| 10(c) | Ex.2(c) | 98 | 96,3 | 0,6 | 2,0 | OUI |
| 11(c) | Ex.3(c) | 98,6 | 97,5 | 0,25 | 1,5 | OUI |
| 12(c) | Ex.4(c) | 97,8 | 98,0 | 0,2 | 0,8 | OUI |
| 13 | Ex.5 | 98,3 | 97,8 | 0,1 | 1,2 | NON |
| 14 | Ex.6 | 98,5 | 97,7 | 0,1 | 1,5 | NON |
| 15 | Ex.7 | 98,5 | 97,2 | 0,1 | 2,0 | NON |
| 16 | Ex.8 | 97,1 | 98,0 | 0,2 | 1,0 | NON |

Les compositions catalytiques des exemples 1 à 4 de comparaison, dont les résultats en oxychloration et en collage sont rapportés dans les exemples 9 à 12, donnent une très bonne sélectivité de l'éthylène en 1,2-dichloréthane mais provoquent du collage sur les parois du réacteur. Par contre, les exemples 13 à 16 démontrent que les compositions selon l'invention permettent d'obtenir un bon rendement en 1,2-dichloréthane par rapport à l'HCl et une très bonne sélectivité en 1,2-dichloréthane sans que des phénomènes de collage ne se manifestent.

## Revendications

1. Composition catalytique d'oxychloration comprenant des chlorures de cuivre, de magnésium et de lithium déposés sur un support inerte, caractérisée en ce qu'elle contient en outre au moins un chlorure de métal alcalin autre que le lithium, en ce qu'elle présente une teneur en cuivre, calculée sous forme métallique par rapport au poids total de la composition catalytique, de 30 à 90 g/kg et en ce que les différents chlorures de métaux sont présents dans des rapports atomiques Cu : Mg : Li : autre(s) métal(aux) alcalin(s) de 1 : 0,1-1,5 : 0,01-1,0 : 0,001-0,8.

2. Composition catalytique selon la revendication 1, caractérisée en ce que le chlorure de métal alcalin est choisi parmi les chlorures de sodium, de potassium et leurs mélanges.

3. Composition catalytique selon la revendication 1, caractérisée en ce que le chlorure de métal alcalin est le chlorure de sodium.

4. Composition catalytique selon la revendication 1, caractérisée en ce que le chlorure de métal alcalin est le chlorure de potassium.

5. Composition catalytique selon la revendication 1, caractérisée en ce que le chlorure de métal alcalin est un mélange de chlorure de sodium et de chlorure de potassium.

6. Composition catalytique selon l'une quelconque des revendications 1 à 5, caractérisée en ce que sa teneur en cuivre est comprise entre 40 et 70 g/kg.

7. Composition catalytique selon l'une quelconque des revendications 1 à 6, caractérisée par des rapports atomiques Cu : Mg : Li : autre(s) métal(aux) alcalin(s) de 1 : 0,3-1,0 : 0,1-0,8 : 0,01-0,6.

8. Composition catalytique selon l'une quelconque des revendications 1 à 6, caractérisée en ce que le rapport atomique entre la somme de tous les métaux alcalins et le cuivre ne dépasse pas 1,5.

9. Composition catalytique selon l'une quelconque des revendications 1 à 8, caractérisée en ce que le support est une alumine.

10. Procédé d'oxychloration de l'éthylène en 1,2-dichloréthane, caractérisé en ce que la réaction d'oxychloration est catalysée par une composition catalytique selon l'une quelconque des revendications 1 à 9.

11. Procédé d'oxychloration selon la revendication 10, caractérisé en ce que la composition catalytique est sous forme de lit fluidisé.

12. Procédé d'oxychloration selon la revendication 10, caractérisé en ce que la réaction d'oxychloration est effectuée en présence d'oxygène pur.

## Claims

1. Catalytic composition for oxychlorination comprising copper chloride, magnesium chloride and lithium chloride deposited on an inert support, characterized in that it contains, in addition, at least one alkali metal chloride other than lithium chloride, in that it possesses a copper content, calculated in the form of the metal relative to the total weight of the catalytic composition, of 30 to 90 g/Kg and in that the different metal chlorides are present in Cu/Mg/Li/other alkali metal(s) atomic ratios of 1:0.1-1.5:0.01-1.0:0.001-0.8.

2. Catalytic composition according to Claim 1, characterized in that the alkali metal chloride is chosen from sodium chloride, from potassium chloride and mixtures thereof.

3. Catalytic composition according to Claim 1, characterized in that the alkali metal chloride is sodium chloride.

4. Catalytic composition according to Claim 1, characterized in that the alkali metal chloride is potassium chloride.

5. Catalytic composition according to Claim 1, characterized in that the alkali metal chloride is a mixture of sodium chloride and potassium chloride.

6. Catalytic composition according to any one of Claims 1 to 5, characterized in that its copper content is between 40 and 70 g/kg.

7. Catalytic composition according to any one of Claims 1 to 6, characterized by Cu/Mg/Li/other alkali metal(s) atomic ratios of 1:0.3-1.0:0.1-0.8:0.01-0.6.

8. Catalytic composition according to any one of Claims 1 to 6, characterized in that the atomic ratio of the sum of all the alkali metals to copper does not exceed 1.5.

9. Catalytic composition according to any one of Claims 1 to 8, characterized in that the support is an alumina.

10. Process for the oxychlorination of ethylene to 1,2-dichloroethane, characterized in that the oxychlorination reaction is catalysed by a catalytic composition according to any one of Claims 1 to 9.

11. Oxychlorination process according to Claim 10, characterized in that the catalytic composition is in the form of a fluidized bed.

12. Oxychlorination process according to Claim 10, characterized in that the oxychlorination reaction is performed in the presence of pure oxygen.

## Patentansprüche

1. Katalytische Zusammensetzung zur Oxychlorierung, die die Chloride von Kupfer, Magnesium und Lithium, die auf einem inerten Träger abgeschieden sind, umfaßt, dadurch gekennzeichnet, daß sie außerdem wenigstens ein von Lithiumchlorid verschiedenes Alkalimetallchlorid enthält, dadurch, daß sie einen Kupfergehalt, berechnet als Metall in Bezug auf das Gesamtgewicht der katalytischen Zusammensetzung, von 30 bis 90 g/kg aufweist, und dadurch, daß die verschiedenen Metallchloride in Atomverhältnissen Cu : Mg : Li : anderes(andere) Alkalimetall(e) von 1 : 0,1-1,5 : 0,01-1,0 : 0,001-0,8 vorhanden sind.

2. Katalytische Zusammensetzung gemäß Anspruch 1, dadurch gekennzeichnet, daß das Alkalimetallchlorid unter den Chloriden von Natrium, Kalium und ihren Gemischen ausgewählt ist.

3. Katalytische Zusammensetzung gemäß Anspruch 1, dadurch gekennzeichnet, daß das Alkalimetallchlorid Natriumchlorid ist.

4. Katalytische Zusammensetzung gemäß Anspruch 1, dadurch gekennzeichnet, daß das Alkalimetallchlorid Kaliumchlorid ist.

5. Katalytische Zusammensetzung gemäß Anspruch 1, dadurch gekennzeichnet, daß das Alkalimetallchlorid ein Gemisch aus Natriumchlorid und Kaliumchlorid ist.

6. Katalytische Zusammensetzung gemäß einem der Ansprüche 1 bis 5, dadurch gekennzeichnet, daß ihr Kupfergehalt zwischen 40 und 70 g/kg beträgt.

7. Katalytische Zusammensetzung gemäß einem der Ansprüche 1 bis 6, gekennzeichnet durch Atomverhältnisse Cu : Mg : Li : anderes(andere) Alkalimetall(e) von 1 : 0,3-1,0 : 0,1-0,8 : 0,01-0,6.

8. Katalytische Zusammensetzung gemäß einem der Ansprüche 1 bis 6, dadurch gekennzeichnet, daß das Atomverhältnis zwischen der Summe aller Alkalimetalle und Kupfer 1,5 nicht übersteigt.

9. Katalytische Zusammensetzung gemäß einem der Ansprüche 1 bis 8, dadurch gekennzeichnet, daß der Träger ein Aluminiumoxid ist.

10. Verfahren zur Oxychlorierung von Ethylen zu 1,2-Dichlorethan, dadurch gekennzeichnet, daß die Oxychlorierungsreaktion durch eine katalytische Zusammensetzung gemäß einem der Ansprüche 1 bis 9 katalysiert wird.

11. Verfahren zur Oxychlorierung gemäß Anspruch 10, dadurch gekennzeichnet, daß die katalytische Zusammensetzung als Fließbett vorliegt.

12. Verfahren zur Oxychlorierung gemäß Anspruch 10, dadurch gekennzeichnet, daß die Oxychlorierungsreaktion in Gegenwart von reinem Sauerstoff ausgeführt wird.
